(19) Europäisches Patentamt
European Patent Office
Office européen des brevets

(11) Publication number: 0 126 348 A2

# (12) EUROPEAN PATENT APPLICATION

(21) Application number: 84104966.1

(22) Date of filing: 03.05.84

(51) Int. Cl.³: A 61 K 9/02, A 61 K 47/00

(30) Priority: 19.05.83 JP 87960/83
24.09.83 JP 176628/83

(43) Date of publication of application: 28.11.84
Bulletin 84/48

(84) Designated Contracting States: AT BE CH DE FR GB IT LI NL SE

(71) Applicant: Kyoto Pharmaceutical Industries, Ltd., 38, Nishinokyo Tsukinowa-cho Nakakyo-ku, Kyoto-shi Kyoto 604 (JP)

(72) Inventor: Mori, Takayoshi, 13-153, Yamada Hamuro-cho Nishikyo-ku, Kyoto-shi Kyoto (JP)
Inventor: Nishimura, Kenichi, 14, Yamanouchi Miyamae-cho Ukyo-ku, Kyoto-shi Kyoto (JP)
Inventor: Kakeya, Nobuharu, 10-16, Takadai 3-chome, Nagaokayo-shi Kyoto (JP)
Inventor: Kitao, Kazuhiko, 12, Sandan Osa-cho Matsugasaki, Sakyo-ku Kyoto-shi Kyoto (JP)

(74) Representative: Barz, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)

(54) **Composition for rectal administration and method of promoting rectal drug absorption.**

(57) A composition for rectal administration which comprises at least one member selected from among fatty acids containing 8–10 carbon atoms and salts thereof, and at least one compound selected from the group consisting of nonionic surfactants, alkali metal salts of inorganic acids, alkali metal salts of carboxylic acids containing two or more carboxyl groups, basic amino acids, sugar amines, and amines of the general formula

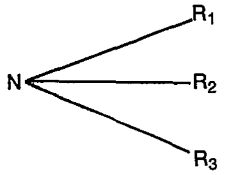

wherein $R_1$, $R_2$ and $R_3$ each is an unsubstituted or substituted alkyl group.

EP 0 126 348 A2

# COMPOSITION FOR RECTAL ADMINISTRATION AND METHOD OF PROMOTING RECTAL DRUG ABSORPTION

This invention relates to a composition for rectal administration and a method of promoting rectal drug absorption.

More particularly, the invention relates to a composition for rectal administration which brings about an improvement in rectal drug absorption and to a method of promoting rectal drug absorption which comprises administering a drug rectally in the presence of a specific compound.

In an earlier patent application (Japanese Patent Application No. Sho 55-149209), the present applicants disclosed that rectal administration of a drug together with a fatty acid containing 8-14 carbon atoms or a salt thereof results in a remarkable improvement in rectal absorption of the drug. As a result of continued research, it was found that a further improvement in rectal drug absorption can be obtained by rectally administering a drug together with a combination of a fatty acid containing 8-10 carbon atoms with a nonionic surfactant, an alkali metal salt of an inorganic acid, an alkali metal salt of a carboxylic acid containing two or more carboxyl groups, a basic amino acid, a sugar amine, or an organic amine of the general formula

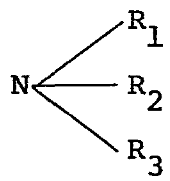

(I)

wherein $R_1$, $R_2$ and $R_3$ each is an unsubstituted or substituted alkyl group. The above finding has led to completion of the present invention.

Thus, the present invention provides a composition for rectal administration which comprises at least one member (component A) selected from among fatty acids containing 8-10 carbon atoms and salts thereof, and at least one member (component B) selected from among nonionic surfactants, alkali metal salts of inorganic acids, alkali metal salts of carboxylic acids containing two or more carboxyl groups, basic amino acids, sugar amines and organic amines of the general formula (I), and a method of promoting rectal drug absorption which comprises rectally administering a drug in the presence of the above components A and B.

As the fatty acids containing 8-10 carbon atoms to be used in accordance with the invention preferred are capric acid and caprylic acid, and the salts include alkali metal salts (e.g. sodium salt, potassium salt), alkaline earth metal salts (e.g. calcium salt) and organic amine salts (e.g. basic amino acid salts, such as lysine salt and arginine salt). Preferred one is the salts of the said fatty acids.

As the nonionic surfactants, those having an HLB value of 5-20 are generally used. Thus, for instance, there may be mentioned polyoxyethylene alkyl ethers [e.g. NIKKOL BL-21(Nikko Chemicals)], polyoxyethylene sorbitan fatty acid esters (e.g. NIKKOL TO-10M), polyethylene glycol fatty acid esters (e.g. NIKKOL MYS-40) and glycerol fatty acid esters (e.g. NIKKOL-MGK). Preferred one is polyoxyethylene alkyl ethers.

The inorganic acid alkali metal salts to be used in accordance with the invention include, among others, alkali metal carbonates (e.g. sodium carbonate), alkali metal hydrogen carbonates (e.g. sodium hydrogen carbonate) and alkali metal phosphates (e.g. trisodium phosphate, disodium hydrogen phosphate). The alkali metal salts of carboxylic acids containing two or more carboxyl groups include alkali metal salts of citric acid (e.g. trisodium citrate), alkali metal salts of tartaric acid (e.g. disodium tartrate) and alkali metal salts of other hydroxy acids and succinic acid (e.g. disodium succinate), amongst others.

The basic amino acids include, among others, lysine and arginine.

The sugar amines include, among others, glucosamine.

Referring to $R_1$, $R_2$ and $R_3$, which are constituents of the organic amine (I) and each is an alkyl group which may optionally be substituted, the alkyl group or alkyl moiety for each of $R_1$, $R_2$ and $R_3$ preferably contains 1-4 carbon atoms and is, for example, methyl, ethyl, propyl, isopropyl or butyl. As the substituent, preferred is a hydroxyl group. Thus, a hydroxyethyl group is an example of the substituted alkyl group.

Examples of the organic amine (I) are triethylamine and triethanolamine.

The composition for rectal administration according to the invention comprises a carrier and the components A and B uniformly dispersed or dissolved in said carrier. As practically preferred examples of the carrier may be mentioned a base composition for manufacturing rectal preparations.

The base composition for manufacturing rectal preparations is *per se* known and includes, among others, oleaginous bases and water-soluble bases. The oleaginous bases are, for example, fats and oils of the vegetable origin, such as cocoa butter, peanut butter, corn oil, fatty acid glycerol esters [e.g. WITEPSOL (Dynamit Noble), SB-Base (Kanegafuchi Chemical Industry), O.D.O. (Nisshin Oil)], and mineral oils, such as vaseline and paraffin. The water-soluble bases are, for example, polyethylene glycol, propylene glycol and glycerin.

It is preferable to further incorporate at least on member selected from among carboxymethylcellulose salts, alginic acid salts, polyacrylic acid salts and carboxyvinyl polymers.

As the carboxymethylcellulose salts, alginic acid salts and polyacrylic acid salts, there are preferred the respective alkali metal salts, especially the sodium salts.

Preferred carboxyvinyl polymers are, for example, HIVISWAKO-130, HIVISWAKO-104 and HIVISWAKO-105 (all available from Wako Pure Chemical Industries).

The composition according to the invention may also contain known ingredients such as an antioxidant, a filler.

The composition according to the invention, when rectally

administered with a drug so far administered rectally, causes a further increase in the rectal absorption of the drug. In addition, the composition makes it possible to rectally administer a drug so far administered by some other route than the rectal route because of poor rectal absorption thereof.

Accordingly, it is preferable to make up a rectal preparation by formulating a drug into the composition according to the invention. Examples of such preparation are the so-called rectal suppository and a soft capsule preparetion manufactured by filling a soft capsule with a suspension or ointment-like composition composed of a base and a drug dispersed therein.

The rectal suppository, for instance, can be prepared by uniformly dispersing the components A and B in a conventional carrier such as base composition, adding a drug, causing the drug to disperse uniformly, filling a suppository container with the resulting composition and thereby effecting molding. The order of addition is not always limited to the above-mentioned one.

As the drug, there may be used any drug which, when absorbed into the blood, is capable of exhibiting its pharmacological or biological activity. The drug preferably has a molecular weight of not more than 12,000, more preferably not more than 6,000. More specifically, the drug is, for example, a β-lactam ring-containing antimicrobial (e.g. a β-lactam antibiotic such as a penicillin or cephalosporin, inclusive of analogs containing an oxygen atom in lieu of the sulfur atom in the penam ring or cephem ring, respectively; clavulanic acid,

thienamycin), an aminoglycoside antibiotic, a polysaccharide drug, a peptide drug or a nucleic acid drug.

The penicillin includes, among others, ampicillin, ciclacillin, cloxacillin, benzylpenicillin, carbenicillin, piperacillin, mezlocillin, pirbenicillin and ticarcillin.

The cephalosporin includes, among others, cephalothin, cefoxitin, cefazolin, cephaloridine, cephacetrile, cefotiam, ceforanide, cefanone, cefaclor, cefadroxil, cefatrizine, cephradine, cephaloglycine, cefatriaxone, cefotetan, latamoxef, cefpiramide, cefoperazone, cefmenoxime, cefotaxime, ceftazidime, ceftizoxime, AC-1370, MT-141 and azthreonam.

The aminoglycoside includes, among others, streptomycin, kanamycin, fradiomycin, paromomycin, bekanamycin, ribostamycin, gentamicin, tobramycin, dibekacin, sisomicin, micronomicin, netilmicin, amikacin, fortimicin, habekacin and other aminoglycoside antibiotics.

The peptide drug includes, among others, insulin, angiotensin, ACTH, TRH and analogs thereof, enkephalins, LH-RH and a peptide antibiotic (e.g. polymyxin B, colistin, gramicidin, bacitracin). The nucleic acid drug includes citicoline.

Among the drugs to be used according to the invention, AC-1370 is 1-[(6R,7R)-2-carboxy-7-[(R)-[2-(5-carboxy-1H-imidazol-4-carboxamido)-2-phenyl)acetamido]-8-oxo-5-thia-1-azabicyclo[4.2.0]oct-2-en-3-yl]methyl-4-(2-sulfoethyl)pyridinium hydroxide (inner salt, monosodium salt) and MT-141 is (6R,7S)-7-[(2S)-2-amino-2-carboxyethylthioacetamido]-7-methoxy-3-[[(1-methyl-1H-tetrazol-5-yl)thio]methyl]-8-oxo-5-thia-1-azabicyclo[4.2.0]oct-2-ene-2-carboxylate (heptahydrate, sodium salt).

The components A and B are incorporated in the composition according to the invention each in an amount sufficient to promote the absorption of the drug to be incorporated later. The amount depends on the carrier used, the drug requiring promotion of absorption, and other factors. Generally, however, the components A and B are used in a total amount of 0.05-20% by weight, preferably 0.1-15% by weight, more preferably 0.2-10% by weight.

As for the ratio between the components A and B, the component B is used generally in an amount of 1-300 parts by weight, preferably 10-200 parts by weight, more preferably 50-150 parts by weight, per 100 parts by weight of the component A.

When a drug is incorporated into the composition according to the invention, the drug is incorporated in an amount sufficient to produce its pharmacological or biological effects. The amount depends on the drug, subject to be treated therewith, symptoms and other factors, and may be selected on the case-by-case basis. For instance, an antibiotic is added in an amount which corresponds to a single dose of 10-500 mg.

The present invention will be more concretely explained by the following Example, but they should not be thought to indicate the scope of the invention.

## Example 1

WITEPSOL H-5 (Dynamit Nobel; 22.4 g) and NIKKOL MYS-40 (1.1 g) were melted at about 60-70°C, 0.84 g of capric acid was added at about 50°C or below, followed by stirring to attain homogeneous dispersion. To the composition thus obtained, 8.15 g of dibekacin sulfate was added, followed by stirring to

attain homogeneous dispersion. The resulting composition was filled into suppository containers in 1.25 g portions. After molding in this way, there were obtained dibekacin sulfate suppositories.

Example 2

By the same manner as in Example 1 except for employing NIKKOL BL-21 instead of NIKKOL MYS-40, there were obtained dibekacin sulfate suppositories.

Example 3

Following the procedure of Example 1, there were obtained rectal suppositories having the compositions given in Tables 1-3. The suppositories were administered rectally to dogs or rabbits fasted for 24 hours. After administration, blood or urine sampling was conducted at times intervals and the drug concentration in blood or urinary drug excretion was estimated by determining the activity concentration by the bioassay method. The results obtained are shown in Tables 1-2.

The test organism used was Bacillus subtilis ATCC 6633.

Table 1

Blood concentrations of aminoglycoside antibiotics in dogs

| Drug * | Base | Additive | AUC (μg.hr/ml) |
|---|---|---|---|
| Dibekacin (100 mg in potency) | WITEPSOL H-5 | None | 0.8 |
| | | NIKKOL MYS-40, 4% | 1.0 |
| | | Na caprate, 3% | 14.4 |
| | | Na caprate, 3%<br>NIKKOL MYS-40, 4% | 27.4 |
| | | Na caprate, 3%<br>NIKKOL BL-21, 4% | 37.0 |
| | | Na caprate, 3%<br>NIKKOL BL-21, 4%<br>Disodium hydrogen phosphate, 1% | 42.0 |
| | | Na caprate, 3%<br>Sodium carbonate, 4% | 24.4 |
| | | Na caprate, 3%<br>NIKKOL MYS-40, 4%<br>Sodium carbonate, 4% | 39.9 |
| Fortimicin (100 mg in potency) | Ditto | None | 1.2 |
| | | Na caprate, 3% | 14.8 |
| | | Na caprate, 3%<br>NIKKOL MYS-40, 4% | 21.7 |
| | | Na caprate, 3%<br>NIKKOL BL-21, 4% | 30.0 |
| | | Na caprate, 3%<br>Sodium carbonate, 2% | 21.7 |
| Amikacin | WITEPSOL H-15 | None | 1.0 |
| | | Na caprate, 3%<br>NIKKOL BL-21, 3% | 17.9 |

| Drug * | Base | Additive | AUC (μg.hr/ml) |
|---|---|---|---|
| Gentamicin | WITEPSOL H-15 | None | 0.9 |
| | | Na caprate, 3%<br>NIKKOL BL-21, 4% | 20.8 |
| Sisomicin | Ditto | None | 1.2 |
| | | Na caprate, 3%<br>NIKKOL BL-21, 3% | 19.8 |
| Cefatriaxone | WITEPSOL H-5 | Na caprate, 3%<br>NIKKOL MYS-40, 4% | 22.2 |
| Cefotetan | Ditto | Na caprate, 3%<br>NIKKOL BL-21, 2% | 20.3 |
| AC-1370 | Ditto | Na caprate, 3%<br>NIKKOL BL-25, 2% | 19.8 |
| MT-141 | Ditto | Na caprate, 3%<br>Sodium carbonate, 4% | 21.8 |
| Ceftazidime | Ditto | Na caprate, 3%<br>Sodium carbonate, 4% | 30.9 |
| | | Na caprate, 3%<br>Sodium carbonate, 4% | 29.8 |
| | | Na caprate, 3%<br>Lysine, 4% | 30.1 |
| | | Na caprate, 3%<br>Na citrate, 4% | 27.6 |
| Azthreonam | Ditto | Na caprate, 3%<br>L-Arginine Na, 4% | 27.4 |
| | | Na caprate, 3%<br>Na tartrate, 4% | 23.8 |

Note: * 100 mg in potency

Na : Sodium

Table 2

Urinary excretion of aminoglycoside antibiotics in rabbits

| Drug.* | Base | Additive | Urinary excretion (0-6 hours) (%) |
|---|---|---|---|
| Amikacin | WITEPSOL H-5 | Na caprate, 3% | 35.0 |
| | | Na caprate, 3%<br>NIKKOL MYS-40, 4% | 56.0 |
| | | Na caprate, 3%<br>Sodium carbonate, 4% | 55.5 |
| Netilmicin | Ditto | Na caprate, 3% | 40.0 |
| | | Na caprate, 3%<br>NIKKOL TO-10M, 4% | 52.6 |
| | | Na caprate, 3%<br>Lysine, 4% | 48.0 |
| Micronomicin | Ditto | Na caprate, 3% | 39.2 |
| | | Na caprate, 3%<br>NIKKOL BL-21, 4% | 50.3 |
| | | Na caprate, 3%<br>Arginine Na, 4% | 48.2 |
| Sisomicin | WITEPSOL H-15 | Na caprate, 2% | 30.8 |
| | | Na caprate, 2%<br>NIKKOL BL-25, 4% | 51.6 |
| | | Na caprate, 3%<br>Trisodium phosphate, 4% | 55.0 |
| Tobramycin | WITEPSOL W-35 | Na caprate, 2% | 41.6 |
| | | Na caprate, 2%<br>NIKKOL BL-9, 4% | 53.8 |
| | | Na caprate, 3%<br>Disodium tatrate, 4%<br>NIKKOL BL-9, 4% | 49.2 |
| Gentamicin | Ditto | Na caprate, 3% | 29.8 |
| | | Glycerol monocaprylate, 2%<br>Na caprate, 3% | 52.0 |
| | | Na caprate, 3%<br>Disodium citrate, 2% | 45.8 |

| Drug * | Base | Additive | Urinary excretion (0-6 hours) (%) |
|---|---|---|---|
| Bekanamycin | WITEPSOL W-35 | Na caprate, 3% | 30.1 |
| | | Glycerol monostearate, 3%<br>Na caprate, 3% | 48.5 |
| | | Na caprate, 3%<br>Disodium phosphate, 3% | 40.0 |

Note : * 100 mg in potency

Na : Sodium

CLAIMS: 

1. A composition for rectal administration which comprises at least one member selected from among fatty acids containing 8-10 carbon atoms and salts thereof, and at least one compound selected from the group consisting of nonionic surfactants, alkali metal salts of inorganic acids, alkali metal salts of carboxylic acids containing two or more carboxyl groups, basic amino acids, sugar amines, and amines of the general formula

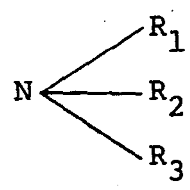

wherein $R_1$, $R_2$ and $R_3$ each is an unsubstituted or substituted alkyl group.

2. A composition for rectal administration as claimed in Claim 1, wherein the compound is a nonionic surfactant having an HLB value of 5-20.

3. A composition for rectal administration as claimed in Claim 2, wherein the nonionic surfactant is a polyoxyethylene alkyl ether.

4. A composition for rectal administration as claimed in Claim 3, wherein the polyoxyethylene alkyl ether is polyoxyethylene(21) lauryl ether.

5. A composition for rectal administration as claimed in Claim 1, wherein the compound is an alkali metal salt of carbonic acid or an alkali metal salt of phosphoric acid.

6. A composition for rectal administration as claimed

in any of Claims 1-5, wherein a drug is incorporated therein.

7. A composition for rectal administration as claimed in any of Claims 1-6, wherein the drug is an aminoglycoside antibiotic.

8. A method of promoting rectal absorption of drugs which comprises administering a drug in the presence of the composition as claimed in any of Claims 1-7.